# EUROPEAN PATENT SPECIFICATION

(11) **EP 4 543 865 B1**
(45) Date of publication and mention of the grant of the patent: **29.04.2026**
(21) Application number: 23734653.1
(22) Date of filing: 22.06.2023
(51) Int. Cl.: C07D 317/24

(54) **PROCESS FOR PREPARING SOLKETAL ACRYLATE BY TRANSESTERIFICATION**
VERFAHREN ZUM HERSTELLEN VON SOLKETALACRYLAT DURCH TRANSESTERIFIKATION
PROCÉDÉ DE PRÉPARATION D'ACRYLATE DE SOLCÉTAL PAR TRANSESTÉRIFICATION

(30) Priority: 24.06.2022 EP 22180937
(43) Date of publication of application: 30.04.2025
(73) Proprietor: BASF SE, 67056 Ludwigshafen am Rhein (DE)
(72) Inventor: MISSKE, Andrea, 67056 Ludwigshafen am Rhein (DE); ELIXMANN, David, 67056 Ludwigshafen am Rhein (DE); WILLERSINN, Stefan, Houston, Texas 77079 (US); FLECKENSTEIN, Christoph, 67056 Ludwigshafen am Rhein (DE); FLEISCHHAKER, Friederike, 67056 Ludwigshafen (DE); EICHHORN, Sabine, 67056 Ludwigshafen (DE)
(74) Representative: BASF IP Association
(86) International application number: PCT/EP2023/067030
(87) International publication number: WO 2023/247725

(56) References cited:
- WO-A1-2016/097840
- WO-A1-2018/146258
- CN-A- 106 008 447

## Description

The invention relates to a process for preparing solketal acrylate by transesterification of solketal with ethyl acrylate.

Solketal acrylate ((2,2-dimethyl-1,3-dioxolan-4-yl)methyl acrylate, IPGA) is particularly useful as a reactive diluent in curable compositions, such as printing inks, preferably inkjet printing inks. Solketal acrylate is an excellent monofunctional monomer acrylate with an outstanding performance profile hardly found for any commercially available monofunctional monomer acrylate in UV inkjet. It combines very low viscosity as pure substance as well as in UV inkjet ink formulations with very high cure speed and very good adhesion on various substrates, such as plastic films.

WO 2018/146258 describes the preparation of (2,2-dimethyl-1,3-dioxolan-4-yl)methyl acrylate (Solketal acrylate, IPGA) 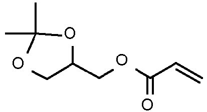 as follows: In a 4L double jacket reactor with column, condenser, reflux splitter, anchor stirrer and lean air introduction ethyl acrylate (2650 g), MeHQ (1.36 g), phenothiazine (136 mg) and solketal (1250 g) were added. Titanium tetra isopropoxylate (54 g) was added, lean air introduction started and the mixture was heated to a sump temperature of 86°C at a vacuum of 800 mbar. A reflux ratio of 10:1 (reflux:distillate) was adjusted when the mixture started to boil which was adapted in the course of the reaction. The sump temperature increased to 104°C while the vacuum was adapted to 630 mbar. After 5 h GC of the distillate showed a content of 0.7% ethanol (GC area%). 300 mL of water were added. After 30 min water and ethyl acrylate were distilled off with a bath temperature of 80°C at a vacuum of 20 mbar. The product was obtained after filtration in 1670 g yield and 96% purity (GC area%).

CN106008447 also discloses a method for preparing the acrylate of 2,2-dimethyl-1,3-dioxolane methanol (solketal) by transesterification with methyl acrylate in the presence of an organotitanium or an organotin catalyst, such tetrabutyl titanate, butyl titanate, tetraisopropyl titanate, dibutyl tin dilaurate, dibutyltin dichloride, tributyl tin acetate, dibutyltin oxide and tributyl tin chloride.

It is an object of the invention to provide a process for preparing solketal acrylate by transesterification of ethyl acrylate with solketal ((2,2-dimethyl-1,3-dioxolan-4-yl)methanol) in which the reaction time or batch time is minimized.

The object is achieved by a process for preparing solketal acrylate by transesterifying ethyl acrylate with solketal, comprising the steps of:
(i) reacting ethyl acrylate with solketal in the presence of a catalyst comprising titanium(IV) or zirconium(IV) and a stabilizer in the presence of ethyl acrylate as azeotropic agent which forms an azeotrope with ethanol,
(ii) continuously distilling off and condensing an azeotrope containing ethyl acrylate and ethanol by means of a distillation column operated under reflux conditions,

wherein steps (i) and (ii) are conducted simultaneously until a solketal conversion of ≥ 95% is reached,
characterized in that step (ii) is carried out by diluting the condensed azeotrope with additional ethyl acrylate and using this mixture as a reflux to the distillation column.

It has been found that, surprisingly, reaction time can be significantly reduced by diluting the condensed azeotrope containing ethyl acrylate and ethanol with essentially pure ethyl acrylate and using this mixture as a reflux to the column as soon as the reaction mixture starts boiling and a distillate containing ethyl acrylate and ethanol is condensed and withdrawn at the top of the column. Essentially pure in the context of the present invention means that the ethyl acrylate has a purity of at least 98 wt.%.

The essentially pure ethyl acrylate may contain up to 2 wt.% of ethanol.

In a preferred embodiment, diluting the condensed azeotrope with ethyl acrylate is carried out by prefilling a distillate collecting vessel with ethyl acrylate and mixing the condensed azeotrope withdrawn from the top of the distillation column with the ethyl acrylate in this vessel. By this means, the condensed azeotrope is continuously diluted with ethyl acrylate before being returned as a reflux to the top of the distillation column.

In general, the ethanol content of the reflux is within the range of from 10 wt.% and 30 wt.% while steps (i) and (ii) are carried out.

In general, the reflux ratio of reflux : distillate in the distillation column is from 10 : 1 to 2 : 1, preferably from 10 : 2 to 3 : 1, more preferably from 4 : 1 to 2 : 1.

The reaction of ethyl acrylate with solketal is effected in the presence of a catalyst comprising titanium(IV) or zirconium(IV). Suitable catalysts comprising titanium(IV) or zirconium(IV) are the Ti(IV) and Zr(IV) tetraalkoxides of linear or branched C₁-C₆ alcohols, preferably the tetraethoxides, tetraisopropoxides, tetrabutoxides and the metalate of the reactant alcohol used or mixtures thereof. Metalates substituted by different alcohols or by acetylacetone are also possible.

The reaction of ethyl acrylate with solketal is additionally effected in the presence of one or more stabilizers (polymerization inhibitors). Examples of suitable stabilizers are N-oxides (nitroxyl or N-oxyl radicals, i.e., compounds bearing at least one N-O group), for example 4-hydroxy-2,2,6,6-tetramethylpiperidine N-oxyl, 4-oxo-2,2,6,6-tetramethylpiperidine N-oxyl, 4-acetoxy-2,2,6,6-tetramethylpiperidine N-oxyl, 2,2,6,6-tetramethylpiperidine N-oxyl, bis(1-oxyl-2,2,6,6-tetramethylpiperidin-4-yl) sebacate, 4,4',4"-tris(2,2,6,6-tetramethylpiperidine N-oxyl) phosphite or 3-oxo-2,2,5,5-tetramethylpyrrolidine N-oxyl; mono- or polyhydric phenols which may bear one or more alkyl groups, for example alkylphenols, for example o-, m- or p-cresol (methylphenol), 2-tert-butylphenol, 4-tert-butylphenol, 2,4-di-tert-butylphenol, 2-methyl-4-tert-butylphenol, 2-tert-butyl-4-methylphenol, 2,6-tert-butyl-4-methylphenol, 4-tert-butyl-2,6-dimethylphenol or 6-tert-butyl-2,4-dimethylphenol; quinones, for example hydroquinone, hydroquinone monomethyl ether, 2-methylhydroquinone or 2,5-di-tert-butylhydroquinone; hydroxyphenols, for example catechol (1,2-dihydroxybenzene) or benzoquinone; aminophenols, for example p-aminophenol; nitrosophenols, for example p-nitrosophenol; alkoxyphenols, for example 2-methoxyphenol (guaiacol, catechol monomethyl ether), 2-ethoxyphenol, 2-iso-propoxyphenol, 4-methoxyphenol (hydroquinone monomethyl ether), mono- or di-tert-butyl-4-methoxyphenol; tocopherols, for example α-tocopherol and 2,3-dihydro-2,2-dimethyl-7-hydroxybenzofuran (2,2-dimethyl-7-hydroxycoumaran), aromatic amines, for example N,N-diphenylamine or N-nitrosodiphenylamine; phenylenediamines, for example N,N'-dialkyl-p-phenylenediamine where the alkyl radicals may be the same or different and each independently consist of 1 to 4 carbon atoms and may be straight-chain or branched, for example N,N'-dimethyl-p-phenylenediamine or N,N'-diethyl-p-phenylenediamine, hydroxylamines, for example N,N-diethylhydroxylamine, imines, for example methyl ethyl imine or methylene violet, sulfonamides, for example N-methyl-4-toluenesulfonamide or N-tert-butyl-4-toluenesulfonamide, oximes such as aldoximes, ketoximes or amide oximes, for example diethyl ketoxime, methyl ethyl ketoxime or salicylaldoxime, phosphorus compounds, for example triphenylphosphine, triphenyl phosphite, triethyl phosphite, hypophosphorous acid or alkyl esters of the phosphorous acids; sulfur compounds, for example diphenyl sulfide or phenothiazine, or mixtures thereof.

Preference is given to hydroquinone, hydroquinone monomethyl ether, phenothiazine, 4-hydroxy-2,2,6,6-tetramethylpiperidine N-oxyl, 4-oxo-2,2,6,6-tetramethylpiperidine N-oxyl, 2-tert-butylphenol, 4-tert-butylphenol, 2,4-di-tert-butylphenol, 2-tert-butyl-4-methylphenol, 6-tert-butyl-2,4-dimethylphenol, 2,6-di-tert-butyl-4-methylphenol and 2-methyl-4-tert-butylphenol and phenothiazine.

Particular preference is given to hydroquinone monomethyl ether (MeHQ), phenothiazine (PTZ) and 4-hydroxy-2,2,6,6-tetramethylpiperidine N-oxyl (HO-TEMPO).

Advantageously, oxygen may additionally be used as a polymerization inhibitor.

For further stabilization, an oxygenous gas, preferably air or a mixture of air and nitrogen (lean air), may be present.

The transesterification reaction (steps (i) and (ii)) is generally carried out at a temperature of from 60°C to 120°C, preferably from 70°C to 110°C. In the course of this, an azeotrope of ethyl acrylate and ethanol is distilled off continuously.

In general, ethyl acrylate is used in a stoichiometric excess. Preferably, the excess of ethyl acrylate per solketal to be esterified is up to 300 wt.%, more preferably up to 200 wt.% and especially from 110 to 150 wt.%.

The catalyst is used in a concentration of 0.1 to10 mol-% based on the amount of solketal used, preferably in a concentration of 0.1 to 5 mol-%.

The transesterification in steps (i) and (ii) may be conducted at atmospheric pressure, but also under elevated pressure or reduced pressure. In general, it is conducted at 100 to 1000 mbar, preferably at 300-800 mbar (atmospheric pressure = 1000 mbar). The reaction time is generally 5 to 120 hours, preferably 12 to 48 hours.

Most preferably, steps (i) and (ii) are carried out at a pressure of from 400 to 600 mbar(a) and a temperature of from 80 to 105 °C.

The reaction may be conducted in all reactors suitable for a reaction of this type. Such reactors are known to those skilled in the art. The reaction is preferably effected in a stirred tank reactor.

The batch can be mixed using any desired apparatuses, for example stirring apparatuses. The mixing can also be effected by feeding in a gas, preferably an oxygen-containing gas.

The ethyl acrylate as the azeotroping agent may be subsequently replenished in the reactor. For this purpose, the azeotropic mixture of ethyl acrylate and ethanol distilled off is stirred with water in a mixing vessel and then transferred into a phase separator, wherein the ethanol dissolves in water and the organic phase separates out as the upper layer. The organic phase can be returned to the reaction mixture. It is alternatively also possible to add fresh ethyl acrylate and work up the ethyl acrylate/ethanol mixture in a separate step or to wholly or partly dispense with replenishment of ethyl acrylate.

In one embodiment, ethanol is removed from the azeotrope of ethyl acrylate and ethanol distilled off in step (ii) by washing with water. The ethyl acrylate may be recycled into the reaction vessel.

Steps (i) and (ii) are conducted until the solketal used has been converted to the acrylate ester to an extent of at least 95%, preferably to an extent of at least 97%.

Preferably, the process of the invention further comprises step (iii):
(iii) evaporating unreacted ethyl acrylate from the product mixture under evaporating conditions when a solketal conversion of ≥ 95% is reached.

In particular step (iii) is carried out when a solketal conversion of ≥ 97% is reached.

Preferably, step (iii) is carried out at a pressure of from 10 to 500 mbar and at a temperature of from 60 to 100 °C.

Preferably, the process of the invention comprises additional step (iv):
(iv) stripping remaining ethyl acrylate form the product mixture with a gas flow.

The gas flow used in step (iv) may be an inert gas flow, such as a nitrogen gas flow, or an oxygen containing gas flow, such as an air flow.

Preferably, step (iv) is carried out until the ethyl acrylate concentration in the product mixture is ≤ 200 ppm.

The product mixture comprising solketal acrylate and the catalyst can be further worked up by subsequent steps (v) to (vii):
(v) adding water to the product mixture which comprises solketal acrylate and the catalyst,
(vi) distilling water out of the product mixture,
(vii) removing the hydrolyzate of the catalyst comprising titanium(IV) or zirconium(IV).

Step (vii) can also be conducted before step (vi). Step (v) can also be carried out before step (iii). Then step (vi) can be performed together with step (iii). Alternatively, steps (v) and (vi) can also be carried out before step (iv).

This distillation (vi) is generally effected at a temperature of 40°C to 100°C, preferably 60°C to 100°C, and a variable pressure of 2 to 700 mbar. The distillative removal can be carried out, for example, in a stirred tank with jacket heating and/or internal heating coils under reduced pressure.

The sparingly soluble hydrolyzate is removed in step (vii), for example, by filtration or centrifugation.

The filtration can be conducted, for example, with a pressure filter. In process engineering terms, for a filtration in the process of the invention, it is possible to use any filtration methods and apparatuses known per se, for example those described in Ullmann's Encyclopedia of Industrial Chemistry, 7th ed., 2013 Electronic Release, chapter: Filtration, 1. Fundamentals and Filtration 2. Equipment. For example, these may be cartridge filters, filter presses, pressure plate filters, bag filters or drum filters. Preference is given to using cartridge filters or pressure plate filters. The filtration can be conducted with or without filtering aids. Suitable filtering aids are filtering aids based on kieselguhr, perlite and cellulose.

Suitable centrifuges and also separators are known to the expert. In process engineering terms, for a centrifugation in the process of the invention, it is possible to use any centrifugation methods and apparatuses known per se, for example those described in Ullmann's Encyclopedia of Industrial Chemistry, 7th ed., 2013 Electronic Release, chapter: Centrifuges, Filtering and Centrifuges, Sedimenting.

The invention is more particularly described by the examples which follow.

### Examples

### Simulation Study

All process simulation studies were carried out with CHEMADIS, a discontinuous process flow sheet simulation tool of BASF SE. The simulation tool predicts the transient behavior over time, based on publicly available physical property databases as well as on own measurements by using well-established thermodynamic models. A reaction kinetics model was derived from lab experiments and also integrated into the process simulation.

### Example 1

In a 14 m³ half-coil jacket reactor with a three-stage cross-arm stirrer topped by a column (Koch-Glitsch IMTP 25 random packing) with 10 theoretical stages, condenser, reflux pump, reflux splitter, distillate drum, vacuum system and air introduction solketal (3322 kg), ethyl acrylate (2200 kg stabilized with 1250 ppm MEHQ and 4386 kg stabilized with 15 ppm MEHQ) and an internal recycling stream of 464 kg (50 wt.-% ethyl acrylate, rest ethanol) were added. Titanium tetra isopropoxylate (88 kg) was added, air introduction was started, and the mixture was heated with 1.5 barg steam to a sump temperature of 85° C at a vacuum of 600 mbar. The distillate drum was prefilled with 2500 kg ethyl acrylate stabilized with 15 ppm MEHQ and a reflux ratio of 4 (reflux:distillate) was adjusted when the mixture started to boil (t = 0 h). When the sump temperature reached 97°C, the vacuum was continuously reduced to hold the sump temperature constant at 97 °C. When the solketal conversion in the sump reached > 98 % (t = 30 h), the reflux was set to 0 and the rest ethyl acrylate was evaporated. When the vacuum reached < 40 mbar (t = 32 h), the evaporation was stopped by stopping the steam supply and the air flow was increased continuously to 8.5 Nm³/h to strip the remaining ethyl acrylate from the sump. When the sump concentration showed an ethyl acrylate concentration of < 100 ppm, stripping was finished, and the reactor was cooled down with cooling water in the jacket. The raw product was obtained in 4416 kg yield and 97 % purity. The non-converted excess ethyl acrylate, which was prefilled in the distillate drum can be used in other processes, where the impurities like isopropanol are also removed.

### Example 2

In a 14 m³ half-coil jacket reactor with a three-stage cross-arm stirrer topped by a column (Koch-Glitsch IMTP 25 random packing) with 10 theoretical stages, condenser, reflux pump, reflux splitter, distillate drum, vacuum system and air introduction solketal (3322 kg), ethyl acrylate (2200 kg stabilized with 1250 ppm MEHQ and 4386 kg stabilized with 15 ppm MEHQ) and an internal recycling stream of 464 kg (50 wt.-% ethyl acrylate, rest ethanol) were added. Titanium tetra isopropoxylate (88 kg) was added, air introduction was started, and the mixture was heated with 1.5 barg steam to a sump temperature of 85° C at a vacuum of 600 mbar. The distillate drum was not prefilled and a reflux ratio of 4 (reflux:distillate) was adjusted when the mixture started to boil (t = 0 h). When the sump temperature reached 97°C, the vacuum was continuously reduced to hold the sump temperature constant at 97 °C. When the solketal conversion in the sump reached > 98 % (t = 35 h), the reflux was set to 0 and the rest ethyl acrylate was evaporated. When the vacuum reached < 40 mbar (t = 37 h), the evaporation was stopped by stopping the steam supply and the air flow was increased continuously to 8.5 Nm³/h to strip the remaining ethyl acrylate from the sump. When the sump concentration showed an ethyl acrylate concentration of < 100 ppm, stripping was finished, and the reactor was cooled down with cooling water in the jacket. The same amount of raw product was obtained with 97 % purity in 5 h more time than the preferred example 1.

### Example 3

In a 14 m³ half-coil jacket reactor with a three-stage cross-arm stirrer topped by a column (Koch-Glitsch IMTP 25 random packing) with 10 theoretical stages, condenser, reflux pump, reflux splitter, distillate drum, vacuum system and air introduction solketal (3322 kg), ethyl acrylate (2200 kg stabilized with 1250 ppm MEHQ and 4386 kg stabilized with 15 ppm MEHQ) and an internal recycling stream of 464 kg (50 wt.-% ethyl acrylate, rest ethanol) were added. Titanium tetra isopropoxylate (88 kg) was added, air introduction was started, and the mixture was heated with 1.5 barg steam to a sump temperature of 85° C at a vacuum of 600 mbar. The distillate drum was not prefilled and a reflux ratio of 4 (reflux:distillate) was adjusted when the mixture started to boil (t = 0 h). When the sump temperature reached 97°C, the vacuum was continuously reduced to hold the sump temperature constant at 97 °C. When the solketal conversion in the sump reached > 98 % (t = 35 h), the reflux ratio was kept constant at 4 and the rest ethyl acrylate was removed in a distillation mode. When the vacuum reached < 40 mbar (t = 48 h), the reflux was set to 0, the steam supply was stopped, and the air flow was increased continuously to 8.5 Nm³/h to strip the remaining ethyl acrylate from the sump. When the sump concentration showed an ethyl acrylate concentration of < 100 ppm, stripping was finished, and the reactor was cooled down with cooling water in the jacket. The raw product was obtained in 4520 kg yield and 98 % purity in 11 h more time than in example 2. Although the production amount is around 100 kg/batch higher due to distillation mode, the annual production is much lower due to a significant higher batch time.

### Example 4

In a 4L double jacket reactor topped by a column (Montz A3-750 packing), cooler, reflux splitter, with a three-stage cross-arm stirrer as well as lean air introduction ethyl acrylate (2000 g), ethyl acrylate ethanol azeotrope from a previous experiment (190 g), MeHQ (1.27 g), as well as solketal (1000 g) were added. The mixture was heated to a sump temperature of 85° C at 600 mbar with lean air introduction, and during heating titanium tetra isopropoxide (11.1 g) was added while stirring.

After boiling began, an azeotrope of ethyl acrylate and ethanol was continuously distilled off with a reflux ratio of 10:1, which was adjusted in the course of the reaction. The sump temperature rose to 100 °C in the course of the reaction with the vacuum being adjusted down to 500 mbar. Distillate and sump samples were taken regularly in order to monitor the course of the reaction. To speed up the reaction, in the course of the reaction catalyst was added incrementally (a total of 15.5 g). After 11.5 h of distillation the yield was >98%.

250 ml water were added to the reaction mixture, stirred for 1 h at a bath temperature of 75°C and then water as well as ethyl acrylate were distilled off. In the course of the distillation the pressure was reduced to 12 mbar.

44g filtering aid Harbolite 900 were added to the reaction mixture and filtered via a pressure filter.

After filtration the product was obtained with 96.3 GC area% purity in a 1161 g yield.

### Example 5

In a 4L double jacket reactor topped by a column (Montz A3-750 packing), cooler, reflux splitter, with a three-stage cross-arm stirrer as well as air introduction ethyl acrylate (2502 g), MeHQ (1,38 g), as well as solketal (1035 g) were added. The mixture was heated to a sump temperature of 70° C at 300 mbar with air introduction, and 443g ethyl acrylate were distilled off. At 600 mbar the sump temperature was increased to 90°C and during heating titanium tetra ethylate (9.8 g) was added. Subsequently an ethyl acrylate ethanol azeotrope was distilled off, beginning with a reflux ratio of 10:3. The reflux ratio was adjusted in the course of the reaction. The sump temperature increased to 95 °C in the course of the reaction, with the vacuum being adjusted up to 500 mbar. Distillate and sump samples were taken regularly in order to monitor the course of the reaction. In order to speed up the reaction, in the course of the reaction catalyst was added incrementally (a total of 13.1 g). After 9.5 h of distillation the yield was >98%. 260 ml water were added to the reaction mixture, stirred for 1 h at a temperature of 80°C and then water as well as ethyl acrylate were distilled off. In the course of the distillation the pressure was reduced to 15 mbar. 46 g filtering aid Harbolite 900 were added to the reaction mixture and filtered via a pressure filter.

After filtration the product was obtained with 95,8 GC area% purity in a 1247 g yield.

### Example 6

In a 4L double jacket reactor topped by a column (Montz A3-750 packing), cooler, reflux splitter, with a three-stage cross-arm stirrer as well as air introduction ethyl acrylate (2000 g), ethyl acrylate ethanol azeotrope from a previous experiment (190 g), MeHQ (1.27 g), as well as solketal (1000 g) were added. The mixture was heated to a sump temperature of 86°C at 600 mbar with air introduction, and during heating titanium tetra ethylate (10.4 g) was added while stirring. After boiling began, an azeotrope of ethyl acrylate and ethanol was continuously distilled off with a reflux ratio of 10:2, which was adjusted in the course of the reaction. The sump temperature rose to 94 °C in the course of the reaction with the vacuum being adjusted down to 500 mbar. Distillate and sump samples were taken regularly in order to monitor the course of the reaction. To speed up the reaction, in the course of the reaction catalyst was added incrementally (a total of 11.7 g). After 10 h of distillation the yield was >98%. At a temperature of 80-100°C pressure was slowly reduced to 40 mbar and ethyl acrylate was distilled off. Subsequently the temperature was kept at 80°C for 5 h more while introducing 2-8 L/h air until the concentration of ethyl acrylate in the solution was < 0.01% (controlled by gas chromatography). 250 ml water were added to the reaction mixture, stirred for 1 h at a temperature of 75°C and then the water was distilled off. In the course of the distillation the pressure was reduced to 12 mbar. 44g filtering aid Harbolite 900 were added to the reaction mixture and filtered via a pressure filter.

After filtration the product was obtained with 96.0 GC area% purity in a 1204 g yield.

## Claims

1. A process for preparing solketal acrylate by transesterifying ethyl acrylate with solketal, comprising the steps of:
(i) reacting ethyl acrylate with solketal in the presence of a catalyst comprising titanium(IV) or zirconium(IV) and a stabilizer in the presence of ethyl acrylate as azeotropic agent which forms an azeotrope with ethanol,
(ii) continuously distilling off and condensing an azeotrope containing ethyl acrylate and ethanol by means of a distillation column operated under reflux conditions, wherein steps (i) and (ii) are conducted simultaneously until a solketal conversion of ≥ 95% is reached,
**characterized in that** step (ii) is carried out by diluting the condensed azeotrope with additional ethyl acrylate and using this mixture as a reflux to the distillation column.

2. The process of claim 1, wherein the ethanol content of the reflux is within the range of from 10 to 30 wt.% while steps (i) and (ii) are carried out.

3. The process of claim 1 or 2, wherein the reflux ratio of reflux : distillate in the distillation column is from 10 : 1 to 2 : 1.

4. The process of any one of claims 1 to 3, wherein steps (i) and (ii) are carried out at a pressure of from 400 to 600 mbar and a temperature of from 80 to 105 °C.

5. The process of any one of claims 1 to 4, comprising step (iii):
(iii) evaporating unreacted ethyl acrylate from the product mixture under evaporating conditions when a solketal conversion of ≥ 95% is reached.

6. The process of claim 5, wherein step (iii) is carried out when a solketal conversion of ≥ 97% is reached.

7. The process of claim 5 or 6, wherein step (iii) is carried out at a pressure of from 10 to 500 mbar and a temperature of from 60 to 100 °C.

8. The process of any one of claims 1 to 7 comprising step (iv):
(iv) stripping remaining ethyl acrylate form the product mixture with a gas flow.

9. The process of claim 8, wherein step (iv) is carried out until the ethyl acrylate concentration in the product mixture is ≤ 200 ppm.

10. The process of any one of claims 1 to 9 comprising steps (v) to (vii):
(v) adding water to the product mixture which comprises solketal acrylate and the catalyst,
(vi) distilling water out of the product mixture,
(vii) removing the hydrolyzate of the catalyst comprising titanium(IV) or zirconium(IV),
wherein step (vii) can also be conducted before step (vi).

11. The process of any one of claims 1 to 9 comprising steps (v) to (vii):
(v) adding water to the product mixture which comprises solketal acrylate and the catalyst,
(vi) distilling water out of the product mixture,
(vii) removing the hydrolyzate of the catalyst comprising titanium(IV) or zirconium(IV),
wherein step (v) is conducted before step (iii).

12. The process of claim 11, wherein step (vi) is conducted together with step (iii).

13. The process of claim 8 or 9 comprising steps (v) to (vii):
(v) adding water to the product mixture which comprises solketal acrylate and the catalyst,
(vi) distilling water out of the product mixture,
(vii) removing the hydrolyzate of the catalyst comprising titanium(IV) or zirconium(IV),
wherein step (v) and step (vi) are conducted before step (iv).

14. The process according to any of claims 1 to 13, wherein the catalyst comprises titanium(IV) tetraisopropoxide.

15. The process according to any of claims 1 to 14, wherein the stabilizer is selected from hydroquinone monomethyl ether and phenothiazine.

## Patentansprüche

1. Verfahren zur Herstellung von Solketalacrylat durch Umesterung von Essigsäureethylester mit Solketal, welches die folgenden Schritte umfasst:
(i) die Umsetzung von Essigsäureethylester mit Solketal in Gegenwart eines Titan(IV) oder Zirkonium(IV) und einen Stabilisator umfassenden Katalysators in Gegenwart von Essigsäureethylester als Azeotropiermittel umfasst, das ein Azeotrop mit Ethanol bildet,
(ii) das kontinuierliche Abdestillieren und Kondensieren eines Essigsäureethylester und Ethanol enthaltenden Azeotrops mittels einer unter Rückflussbedingungen betriebenen Destillationskolonne, wobei die Schritte (i) und (ii) gleichzeitig durchgeführt werden, bis eine Solketalumwandlung von ≥ 95 % erreicht ist,
**dadurch gekennzeichnet, dass** man Schritt (ii) durchführt, indem man das kondensierte Azeotrop mit zusätzlichem Essigsäureethylester verdünnt und dieses Gemisch als Rückfluss zur Destillationskolonne verwendet.

2. Verfahren nach Anspruch 1, wobei der Ethanolgehalt des Rückflusses bei der Durchführung der Schritte (i) und (ii) im Bereich von 10 bis 30 Gew.-% liegt.

3. Verfahren nach Anspruch 1 oder 2, wobei das Rückflussverhältnis von Rückfluss : Destillat in der Destillationskolonne 10 : 1 bis 2 : 1 beträgt.

4. Verfahren nach einem der Ansprüche 1 bis 3, wobei die Schritte (i) und (ii) bei einem Druck von 400 bis 600 mbar und einer Temperatur von 80 bis 105 °C durchgeführt werden.

5. Verfahren nach einem der Ansprüche 1 bis 4, welches Schritt (iii) umfasst:
(iii) das Abdampfen von nicht umgesetztem Essigsäureethylester aus dem Produktgemisch unter Verdampfungsbedingungen beim Erreichen einer Solketalumwandlung von ≥ 95 %.

6. Verfahren nach Anspruch 5, wobei Schritt (iii) durchgeführt wird, wenn eine Solketalumwandlung von ≥ 97 % erreicht ist.

7. Verfahren nach Anspruch 5 oder 6, wobei Schritt (iii) bei einem Druck von 10 bis 500 mbar und einer Temperatur von 60 bis 100 °C durchgeführt wird.

8. Verfahren nach einem der Ansprüche 1 bis 7, welches Schritt (iv) umfasst:
(iv) das Abstreifen von verbliebenem Essigsäureethylester aus dem Produktgemisch mit einem Gasstrom.

9. Verfahren nach Anspruch 8, wobei Schritt (iv) durchgeführt wird, bis die Essigsäureethylesterkonzentration in der Produktmischung ≤ 200 ppm beträgt.

10. Verfahren nach einem der Ansprüche 1 bis 9, welches die Schritte (v) bis (vii) umfasst:
(v) die Zugabe von Wasser zu dem Solketalacrylat und den Katalysator umfassenden Produktgemisch,
(vi) das Abdestillieren von Wasser aus dem Produktgemisch,
(vii) das Entfernen des Hydrolysats des Titan(IV) oder Zirkonium(IV) umfassenden Katalysators,
wobei Schritt (vii) auch vor Schritt (vi) durchgeführt werden kann.

11. Verfahren nach einem der Ansprüche 1 bis 9, welches die Schritte (v) bis (vii) umfasst:
(v) die Zugabe von Wasser zu dem Solketalacrylat und den Katalysator umfassenden Produktgemisch,
(vi) das Abdestillieren von Wasser aus dem Produktgemisch,
(vii) das Entfernen des Hydrolysats des Titan(IV) oder Zirkonium(IV) umfassenden Katalysators,
wobei Schritt (v) vor Schritt (iii) durchgeführt wird.

12. Verfahren nach Anspruch 11, wobei Schritt (vi) zusammen mit Schritt (iii) durchgeführt wird.

13. Verfahren nach Anspruch 8 oder 9, welches die Schritte (v) bis (vii) umfasst:
(v) die Zugabe von Wasser zu dem Solketalacrylat und den Katalysator umfassenden Produktgemisch,
(vi) das Abdestillieren von Wasser aus dem Produktgemisch,
(vii) das Entfernen des Hydrolysats des Titan(IV) oder Zirkonium(IV) umfassenden Katalysators,
wobei Schritt (v) und Schritt (vi) vor Schritt (iv) durchgeführt werden.

14. Verfahren nach einem der Ansprüche 1 bis 13, wobei der Katalysator Titan(IV)-tetraisopropanolat umfasst.

15. Verfahren nach einem der Ansprüche 1 bis 14, wobei der Stabilisator aus Hydrochinonmonomethylether und Phenothiazin ausgewählt ist.

## Revendications

1. Procédé de préparation d'acrylate de solketal par transestérification de l'acrylate d'éthyle avec du solketal comprenant les étapes de :
(i) mise en réaction de l'acrylate d'éthyle avec du solketal en présence d'un catalyseur comprenant du titane (IV) ou du zirconium (IV) et un stabilisant en présence d'acrylate d'éthyle comme agent azéotropique formant un azéotrope avec l'éthanol,
(ii) distillation et condensation en continu d'un azéotrope contenant de l'acrylate d'éthyle et de l'éthanol au moyen d'une colonne de distillation exploitée dans des conditions de reflux, dans lequel les étapes (i) et (ii) sont conduites simultanément jusqu'à atteindre une conversion de solketal de ≥ 95 %,
**caractérisé en ce que** l'étape (ii) est réalisée en diluant l'azéotrope condensé avec de l'acrylate d'éthyle additionnel et en utilisant ce mélange comme un reflux vers la colonne de distillation.

2. Procédé selon la revendication 1, dans lequel la teneur en éthanol du reflux se situe dans la plage de 10 à 30 % en poids tandis que les étapes (i) et (ii) sont effectuées.

3. Procédé selon la revendication 1 ou 2, dans lequel le rapport de reflux reflux : distillat dans la colonne de distillation est de 10 : 1 à 2 : 1.

4. Procédé selon l'une quelconque des revendications 1 à 3, dans lequel les étapes (i) et (ii) sont réalisées à une pression de 400 à 600 mbars et à une température de 80 à 105 °C.

5. Procédé selon l'une quelconque des revendications 1 à 4, comprenant l'étape (iii) :
(iii) évaporation d'acrylate d'éthyle n'ayant pas réagi du mélange de produits dans des conditions d'évaporation lorsqu'une conversion de solketal de ≥ 95 % est atteinte.

6. Procédé selon la revendication 5, dans lequel l'étape (iii) est effectuée lorsqu'une conversion de solketal de ≥ 97 % est atteinte.

7. Procédé selon la revendication 5 ou 6, dans lequel l'étape (iii) est effectuée à une pression de 10 à 500 mbars et à une température de 60 à 100 °C.

8. Procédé selon l'une quelconque des revendications 1 à 7, comprenant l'étape (iv) :
(iv) strippage d'acrylate d'éthyle restant du mélange de produits par un flux de gaz.

9. Procédé selon la revendication 8, dans lequel l'étape (iv) est effectuée jusqu'à ce que la concentration en acrylate d'éthyle dans le mélange de produits soit ≤ 200 ppm.

10. Procédé selon l'une quelconque des revendications 1 à 9, comprenant les étapes (v) à (vii) :
(v) ajout d'eau au mélange de produits qui comprend l'acrylate de solketal et le catalyseur,
(vi) distillation d'eau hors du mélange de produits,
(vii) élimination de l'hydrolysat du catalyseur comprenant du titane (IV) ou du zirconium (IV),
dans lequel l'étape (vii) peut également être conduite avant l'étape (vi).

11. Procédé selon l'une quelconque des revendications 1 à 9, comprenant les étapes (v) à (vii) :
(v) ajout d'eau au mélange de produits qui comprend l'acrylate de solketal et le catalyseur,
(vi) distillation d'eau hors du mélange de produits,
(vii) élimination de l'hydrolysat du catalyseur comprenant du titane (IV) ou du zirconium (IV),
dans lequel l'étape (v) est conduite avant l'étape (iii).

12. Procédé selon la revendication 11, dans lequel l'étape (vi) est conduite conjointement avec l'étape (iii).

13. Procédé de la revendication 8 ou 9 comprenant les étapes (v) à (vii) :
(v) ajout d'eau au mélange de produits qui comprend l'acrylate de solketal et le catalyseur,
(vi) distillation d'eau hors du mélange de produits,
(vii) élimination de l'hydrolysat du catalyseur comprenant du titane (IV) ou du zirconium (IV),
dans lequel l'étape (v) et l'étape (vi) sont conduites avant l'étape (iv).

14. Procédé selon l'une quelconque des revendications 1 à 13, dans lequel le catalyseur comprend du tétraisopropoxyde de titane(IV).

15. Procédé selon l'une quelconque des revendications 1 à 14, dans lequel le stabilisant est choisi parmi l'éther monométhylique d'hydroquinone et la phénothiazine.
